# EUROPÄISCHE PATENTSCHRIFT

(11) **EP 1 106 247 B1**
(45) Veröffentlichungstag und Bekanntmachung des Hinweises auf die Patenterteilung: **19.03.2003**
(21) Anmeldenummer: 01101510.4
(22) Anmeldetag: 18.01.1996
(51) Int. Cl.: B01J 23/52, B01J 23/58, C07C 67/055

(54) **Katalysator, Verfahren zu seiner Herstellung und seine Verwendung für die Produktion von Vinylacetatmonomer**
Catalyst,its preparation process and its use to produce vinyl acetate monomer
Catalyseur,procédé pour sa préparation et son utilisation pour la production de monomères d'acétate de vinyle

(30) Priorität: 23.01.1995 DE 19501891
(43) Veröffentlichungstag der Anmeldung: 13.06.2001
(62) Teilanmeldung aus: 96100677.2
(73) Patentinhaber: Degussa-Hüls Aktiengesellschaft, 60287 Frankfurt am Main (DE)
(72) Erfinder: Tacke, Thomas, Dr., 42001 Paducah, KY (US); Ohlrogge, Uwe, Dr., 63456 Hanau-Steinheim (DE); Müller, Herbert, Dr., 67122 Altrip (DE); Lansink-Rotgerink, Hermanus Gerhardus Jozef, Dr., 63776 Mömbris-Mensengesäss (DE); Krause, Helmfried, 63517 Rodenbach (DE); Daly, Francis P., 42071 Murray, KY (US)

(56) Entgegenhaltungen:
- EP-A- 0 431 478
- US-A- 3 822 308
- US-A- 3 939 199
- US-A- 4 087 622
- US-A- 4 321 409

## Beschreibung

Die vorliegende Erfindung betrifft einen Trägerkatalysator für die Produktion von Vinylacetatmonomer (VAM), welcher auf einem Träger aus Siliziumdioxid, Alumosilikat oder Aluminiumoxid als katalytisch aktive Komponenten Palladium, Gold und Alkaliverbindungen enthält, sowie ein Verfahren zu seiner Herstellung und seine Verwendung.

Gold, Palladium und Alkaliverbindungen enthaltende Trägerkatalysatoren werden für die Produktion von Vinylacetat verwendet. Dazu werden Ethen, Essigsäure und molekularer Sauerstoff bzw. Luft in der Gasphase, gegebenenfalls unter Zusatz von Inertgasen, bei Temperaturen zwischen 100 und 250°C und gewöhnlichem oder erhöhtem Druck in Gegenwart des Trägerkatalysators zur Reaktion gebracht.

Ein solches Produktionsverfahren wird in den Patentschriften DE 16 68 088 und US 4,048,096 beschrieben. Diese Patentschriften offenbaren auch ein Verfahren zur Herstellung der Gold, Palladium und Alkaliverbindungen enthaltenden Trägerkatalysatoren. Je nach Ausführungsform werden Katalysatoren mit homogener Edelmetallverteilung über den Trägerquerschnitt und mit mehr oder weniger ausgeprägtem Schalenpröfil erhalten.

Diese Katalysatoren werden gewöhnlich durch Imprägnieren der Träger mit einer basischen Lösung und einer Gold- und Palladiumsalze enthaltenden Lösung erhalten, wobei die Imprägnierungen gleichzeitig oder nacheinander, mit oder ohne Zwischentrocknung erfolgen. Anschließend wird der Träger zur Entfernung gegebenenfalls vorhandener Chloridanteile gewaschen. Vor oder nach dem Waschen werden die auf dem Träger ausgefällten unlöslichen Edelmetallverbindungen reduziert. Die so erhaltene Katalysatorvorstufe wird getrocknet und zur Aktivierung des Katalysators mit Alkaliacetaten oder Alkaliverbindungen imprägniert, die sich unter den Reaktionsbedingungen bei der Produktion von Vinylacetatmonomer ganz oder teilweise in Alkaliacetate umwandeln. Bevorzugte Alkaliverbindungen sind Kaliumverbindungen, insbesondere Kaliumacetat.

Die Reduktion des Katalysators kann in der wäßrigen Phase oder in der Gasphase vorgenommen werden. Zur Reduktion in der wäßrigen Phase eignen sich zum Beispiel Formaldehyd oder Hydrazin. Die Reduktion in der Gasphase kann mit Wasserstoff beziehungsweise Formiergas (95 Vol.-% N₂ + 5 Vol.-% H₂) oder Ethen vorgenommen werden. Gemäß der EP 0 634 209 erfolgt die Reduktion mit Wasserstoff bei Temperaturen zwischen 40 und 260°C, bevorzugt zwischen 70 und 200°C. Häufig wird der Katalysator jedoch erst nach der Aktivierung mit Alkaliacetat direkt im Produktionsreaktor mit Ethen reduziert.

Im Produktionsprozeß wird der Katalysator erst langsam mit den Reaktanden belastet. Während dieser Anfahrphase erhöht sich die Aktivität des Katalysators und erreicht gewöhnlich erst nach Tagen oder Wochen ihr endgültiges Niveau.

Das Dokument EP-A 0431478 beschreibt ein Verfahren zur Herstellung von Vinylacetat in der Gasphase aus Ethylen, Essigsäure und Sauerstoff an einem Katalysator, der Palladium und Gold auf einem Träger enthält. Der Träger besteht aus SiO₂ oder SiO₂ / Al₂O₃-Gemisch, wobei die Trägerteilchen mit Hilfe von Li-, Mg-, Al-, Zn- oder Mn-Salzen einer C₂-C₂₀-Carboxylsäure als Bindemittel gepreßt werden.

Aufgabe der vorliegenden Erfindung ist es, einen Trägerkatalysator für die Produktion von Vinylacetatmonomer anzugeben, welcher bei gleicher beziehungsweise verbesserter Selektivität eine höhere Aktivität als konventionelle Katalysatoren aufweist. Weiterhin soll ein Verfahren zur Herstellung des Katalysators angegeben werden.

Trägerkatalysator für die Produktion von Vinylacetatmonomer, enthaltend auf einem Träger aus Siliziumdioxid, Alumosilikat oder Aluminiumoxid als katalytisch aktive Komponenten Palladium, Gold und Alkaliacetat, erhältlich durch Imprägnieren des Trägers mit einer basischen Lösung, einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile, und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, welcher dadurch gekennzeichnet ist,
daß die Reduktion im Formiergas bei Temperaturen zwischen 300 und 550, bevorzugt zwischen 350 und 500°C durchgeführt wird.

Ein weiterer gegenstand der Erfindung ist ein Verfahren zur Herstellung eines Trägerkatalysators für die Produktion von Vinylacetatmonomer enthaltend auf einem Träger aus Siliziumdioxid, Alumosilikat oder Aluminiumoxid als katalytisch aktive Komponenten Palladium, Gold und Alkaliacetat durch Imprägnieren des Trägers mit einer basischen Lösung, einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile, und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, welches
dadurch **gekennzeichnet** ist,
daß die Reduktion im Formiergas bei Temperaturen zwischen 300 und 550, bevorzugt zwischen 350 und 500°C durchgeführt wird.

Als Trägermaterial für den Katalysator eignen sich Siliziumdioxid, Alumosilikate oder Aluminiumoxid. Bei Siliziumdioxid kann es sich um gefällte oder auch durch Flammenhydrolyse von Siliziumtetrachlorid erhaltene sogenannte pyrogene Kieselsäure handeln. Wichtig ist, daß die Katalysatorträger unter den Reaktionsbedingungen des katalytischen Prozesses, insbesondere unter dem Einfluß von Essigsäure, ihre mechanische Festigkeit behalten. Vorteilhaft sind solche Trägermaterialien, die eine spezifische Oberfläche größer als 10 m²/g aufweisen, insbesondere eine spezifische Oberfläche von 50 - 250 m²/g.

Die Katalysatorträger können als Strangpreßlinge, Tabletten, Ringe oder in anderen für Festbettkatalysatoren üblichen Formen vorliegen. Die äußeren Abmessungen liegen gewöhnlich im Bereich zwischen 2 und 15 mm. Im Falle von pyrogener Kieselsäure eignen sich zum Beispiel die in der DE 38 03 895 C1 und DE 39 12 504 A1 beschriebenen Preßlinge.

Nach Trocknen und Calcinieren der vorbehandelten Katalysatorträger werden sie mit einer Palladium und Gold enthaltenden Lösung imprägniert. Gleichzeitig mit der edelmetallhaltigen Lösung oder in beliebiger Reihenfolge nacheinander werden die Katalysatorträger mit einer basischen Lösung imprägniert, welche ein oder mehrere basische Verbindungen enthalten kann. Die basische Verbindung oder Verbindungen dienen zur Überführung des Palladiums und Golds in ihre Hydroxide.

Die Verbindungen in der basischen Lösung können aus Alkalihydroxiden, Alkalibicarbonaten, Alkalicarbonaten, Alkalisilikaten oder Mischungen davon bestehen. Bevorzugt werden Kaliumhydroxid und Natriumhydroxid verwendet.

Zur Herstellung der edelmetallhaltigen Lösung können als Palladiumsalze beispielsweise Palladiumchlorid, Natrium- bzw. Kaliumpalladiumchlorid oder Palladiumnitrat verwendet werden. Als Goldsalze eignen sich Gold(III)-chlorid und Tetrachlorogold(III)-säure. Vorzugsweise wird von Kaliumpalladiumchlorid beziehungsweise Natriumpalladiumchlorid und Tetrachlorogoldsäure ausgegangen.

Das Imprägnieren des Trägers mit der basischen Lösung beeinflußt die Abscheidung der Edelmetalle im Trägermaterial. Die basische Lösung kann entweder gleichzeitig mit der Edelmetallösung oder in beliebiger Reihenfolge mit dieser Lösung mit dem Katalysatorträger in Kontakt gebracht werden. Bei aufeinanderfolgender Imprägnierung des Trägers mit den beiden Lösungen kann nach dem ersten Imprägnierschritt eine Zwischentrocknung vorgenommen werden.

Bevorzugt werden die Katalysatorträger zuerst mit der basischen Verbindung imprägniert. Die anschließende Imprägnierung mit der Palladium und Gold enthaltenden Lösung führt zur Ausfällung von Palladium und Gold in einer oberflächlichen Schale auf den Katalysatorträgern. Die umgekehrte Reihenfolge der Imprägnierungen führt im allgemeinen zu einer mehr oder weniger homogenen Verteilung der Edelmetalle über den Querschnitt der Katalysatorträger. Bei geeigneter Verfahrensführung können jedoch auch bei umgekehrter Imprägnier-Reihenfolge Katalysatoren mit definierter Schale erhalten werden (siehe z. B. US 4,048,096). Katalysatoren mit homogener oder nahezu homogener Edelmetall-Verteilung weisen im allgemeinen eine geringere Aktivität und Selektivität auf.

Katalysatoren mit Schalendicken von unter 1 mm, bevorzugt von etwa unter 0,5 mm, sind besonders geeignet. Die Schalendicke wird durch die Menge der auf das Trägermaterial aufgebrachten basischen Verbindung relativ zur gewünschten Menge der Edelmetalle beeinflußt. Je höher dieses Verhältnis ist, desto geringer wird die Dicke der sich ausbildenden Schale. Das für eine gewünschte Schalendicke erforderliche Mengenverhältnis von basischer Verbindung zu den Edelmetallverbindungen hängt von der Beschaffenheit des Trägermaterials sowie von der gewählten basischen Verbindung und den Edelmetallverbindungen ab. Das erforderliche Mengenverhältnis wird zweckmäßigerweise durch wenige Vorversuche ermittelt. Die sich ergebende Schalendicke kann dabei in einfacher Weise durch Aufschneiden der Katalysatorteilchen ermittelt werden.

Die minimal notwendige Menge der basischen Verbindung ergibt sich aus der stöchiometrisch berechneten Menge an Hydroxidionen, die zur Überführung des Palladiums und Golds in die Hydroxide benötigt werden. Als Richtwert gilt, daß die basische Verbindung für eine Schalendicke von 0,5 mm in einem 1 bis 10-fachen stöchiometrischen Überschuß angewendet werden muß.

Die Katalysatorträger werden nach dem Verfahren der Porenvolumenimprägnierung mit den basischen Verbindungen und den Edelmetallsalzen belegt. Wird mit Zwischentrocknung gearbeitet, wählt man die Volumina der beiden Lösungen so, daß sie jeweils etwa 90 bis 100 % der Aufnahmekapazität des Trägermaterials entsprechen. Wird auf die Zwischentrocknung verzichtet, so muß die Summe der Einzelvolumina der beiden Imprägnierlösungen der obigen Bedingung entsprechen, wobei die Einzelvolumina im Verhältnis von 1 : 9 bis 9 : 1 zueinander stehen können. Bevorzugt wird ein VolumenVerhältnis von 3 : 7 bis 7 : 3, insbesondere von 1 : 1, angewendet. Als Lösungsmittel wird in beiden Fällen bevorzugt Wasser verwendet. Es können aber auch geeignete organische oder wäßrig-organische Lösungsmittel eingesetzt werden.

Die Umsetzung der Edelmetallsalzlösung mit der basischen Lösung zu unlöslichen Edelmetallverbindungen erfolgt langsam und ist je nach Präparationsmethode im allgemeinen erst nach 1 bis 24 Stunden abgeschlossen. Danach werden die wasserunlöslichen Edelmetallverbindungen mit Reduktionsmitteln behandelt. Eine Gasphasenreduktion mit Formiergas wird angewendet.

Vor beziehungsweise nach der Reduktion der Edelmetallverbindungen wird das auf dem Träger gegebenenfalls vorhandene Chlorid durch eine gründliche Waschung entfernt. Nach der Waschung sollte der Katalysator weniger als 500, besser weniger als 200 ppm, Chlorid enthalten.

Die nach der Reduktion erhaltene Katalysatorvorstufe wird getrocknet und abschließend mit Alkaliacetaten oder Alkaliverbindungen imprägniert, die sich unter den Reaktionsbedingungen bei der Produktion von Vinylacetatmonomer ganz oder teilweise in Alkaliacetate umwandeln. Vorzugsweise wird mit Kaliumacetat imprägniert. Hierbei wird wieder bevorzugt die Porenvolumenimprägnierung angewendet, das heißt die benötigte Menge Kaliumacetat wird in einem Lösungsmittel, vorzugsweise Wasser, gelöst, dessen Volumen etwa der Aufnahmekapazität des vorgelegten Trägermaterials für das gewählte Lösungsmittel entspricht. Dieses Volumen ist etwa gleich dem Gesamtporenvolumen des Trägermaterials.

Der fertige Katalysator wird anschließend bis auf eine Restfeuchte von weniger als 2 % getrocknet. Die Trocknung kann an Luft, gegebenenfalls auch unter Stickstoff als Inertgas, erfolgen.

Für die Synthese von Vinylacetatmonomer ist es zweckmäßig, den Katalysator mit 0,3 bis 4, vorzugsweise 0,5 bis 3 Gew.-% Palladium, 0,1 bis 2, vorzugsweise 0,2 bis 1,5 Gew.-% Gold und 1 bis 10, vorzugsweise 3,5 bis 10 Gew.-% Kaliumacetat, jeweils bezogen auf das Gewicht des eingesetzten Trägers, zu belegen. Im Fall von Katalysatorträgern mit einer Schüttdichte von 500 g/l entsprechen diese Konzentrationsangaben volumenbezogenen Konzentrationen von 1,5 bis 20 g/l Palladium, 0,5 bis 10 g/l Gold und 5 bis 50 g/l Kaliumacetat. Zur Anfertigung der Imprägnierlösungen werden die entsprechenden Mengen der Palladium- und Goldverbindungen in einem Volumen Wasser gelöst, welches etwa 90 bis 100 % der Wasseraufnahmekapazität des vorgelegten Trägermaterials entspricht.

Ebenso wird bei der Anfertigung der basischen Lösung verfahren.

Zur erfindungsgemäßen Vorbehandlung der Katalysatorträger mit den genannten Kationen wird ebenfalls die Porenvolumenimprägnierung eingesetzt. Danach werden die Katalysatorträger bei erhöhten Temperaturen getrocknet und bei Temperaturen von 160 bis 800, bevorzugt 170 bis 700°C, calciniert.

Die auf vorbehandelten Trägern aufgebrachten Katalysatoren weisen eine höhere Aktivität bei der Herstellung von Vinylacetat als konventionelle Katalysatoren auf. Darüber hinaus wird bei den vorbehandelten Katalysatoren eine verbesserte Edelmetallhaftung beobachtet. Bei Verwendung von nicht vorbehandelten Katalysatorträgern treten beim Auswaschen der Chloride nach der Reduktion der Edelmetallverbindungen Edelmetallverluste auf. Diese Verluste betragen für Gold typischerweise etwa 10 % und für Palladium etwa 6 %. Demgegenüber verringern sich die Goldverluste auf ca. 6 % und die Palladiumverluste auf nur ca. 3 %, wenn die Katalysatorträger erfindungsgemäß vorbehandelt werden.

Überraschenderweise wird bei den erfindungsgemäßen Katalysatoren auch eine erhöhte Einzelkornfestigkeit beobachtet. Sie liegt bei den erfindungsgemäßen Trägerkatalysatoren bei 70 N (Beispiel 2) beziehungsweise 78 N (Beispiel 1), während die konventionell hergestellten Trägerkatalysatoren nur eine Einzelkornfestigkeit von etwa 48 N aufweisen (Vergleichsbeispiel 1).

Auch die Edelmetalldispersion ist bei den erfindungsgemäßen Katalysatoren verbessert. So wird zum Beispiel bei den erfindungsgemäßen Katalysatoren ein CO-Wert (CO-Adsorption durch CO-Puls-Chemiesorption) als Maß für die Edelmetalldispersion von 0,186 ml CO/g Katalysator (Beispiel 1) gemessen, während der CO-Wert bei konventionellen Katalysatoren nur bei 0,158 ml CO/g Katalysator (Vergleichsbeispiel 1) liegt. Die Ursache für die verbesserte Dispersion der katalytisch aktiven Edelmetalle könnte die Beobachtung sein, daß die spezifische Oberfläche der erfindungsgemäß vorbehandelten Katalysatorträger sich durch die Imprägnierung mit der basischen Lösung, insbesondere mit Natronlauge oder Kalilauge, nicht verringert. Dagegen zeigen nicht vorbehandelte Träger bei Einwirkung der basischen Lösung eine zum Teil drastische Verminderung der spezifischen Oberfläche. Damit verbunden ist eine entsprechende Erniedrigung der Edelmetalldispersion der Katalysatoren.

Gemäß Erfindung wird der Katalysator in der Gasphase mit Formiergas bei erhöhten Temperaturen reduziert. Die Gasphasenreduktion von Katalysatoren für die Synthese von Vinylacetatmonomer ist zwar schon bekannt. Sie wird jedoch bei relativ niedrigen Temperaturen vorgenommen. So schreibt zum Beispiel die US 4,370,492 eine Reduktion bei Temperaturen zwischen 40 und 260, bevorzugt zwischen 70 und 200°C, vor.

Überraschenderweise zeigt sich, daß Katalysatoren, die gemäß Erfindung im Temperaturbereich zwischen 300 und 550, vorzugsweise zwischen 350 und 500°C mit Formiergas reduziert werden, deutlich höhere Aktivitäten für die Synthese von Vinylacetatmonomer bei gleichzeitig erhöhter Selektivität aufweisen. Dies gilt insbesondere auch für Katalysatoren, deren Träger nicht mit Kationen vorbehandelt wurden.

Ein weiterer Gegenstand der Erfindung ist die Verwendung des erfindungsgemäßen Trägerkatalysators für die Produktion von Vinylacetatmonomer durch Umsetzung von Ethylen, Essigsäure und Sauerstoff an dem Katalysator in der Gasphase bei erhöhtem Druck.

### Vergleichsbeispiel 1 :

Es wurde ein konventioneller Palladium-Gold-Kaliumacetat-Katalysator auf dem Alumosilikatträger KA 160 hergestellt. Die Konzentration der Imprägierlösungen wurde so gewählt, daß der fertige Katalysator 3,3 g Palladium, 1,5 g Gold und 30 g Kaliumacetat pro Liter Schüttvolumen des Katalysatorträgers enthielt. Dies entsprach einer Konzentration von 0,55 Gew.-% Palladium, 0,25 Gew.-% Gold und 5,0 Gew.-% Kaliumacetat, bezogen auf das Gewicht des eingesetzten Trägers.

In einem ersten Schritt wurde der Träger zunächst mit einer basischen Lösung aus Natriumhydroxid in Wasser imprägniert. Das Volumen der wäßrigen NaOH-Lösung entsprach 50 Prozent der Wasseraufnahme des trockenen Trägers. Nach der Imprägnierung mit Natriumhydroxid wurde der Träger unmittelbar ohne Zwischentrocknung mit einer wäßrigen Edelmetallösung aus Natriumpalladiumchlorid und Tetrachlorogoldsäure imprägniert, deren Volumen ebenfalls 50 % der Wasseraufnahmekapazität des trockenen Trägermaterials entsprach.

Nach einer Wartezeit von 1,5 Stunden zwecks Hydrolyse der Edelmetallverbindungen wurden die Trägerteilchen chlorfrei gewaschen.

Der Katalysator wurde getrocknet und wie in der EP 0 634 209 beschrieben bei 150°C in der Gasphase mit Formiergas reduziert.. Danach wurde der Katalysator mit einer wäßrigen Kaliumacetat-Lösung imprägniert und erneut getrocknet. Die Trocknung wurde in der Gasphase mit Stickstoff durchgeführt.

Die Konzentration der basischen Lösung an Natriumhydroxid war so bemessen, daß sich auf den Trägerteilchen eine edelmetallhaltige Schale von 0,3 mm Dicke ausbildete.

### Vergleichsbeispiel 2:

Es wurde ein Palladium-Gold-Kaliumacetat-Katalysator wie im Vergleichsbeispiel 1 beschrieben hergestellt. Als Katalysatorträger wurde der Alumosilikatträger KA 160 verwendet. Der Katalysator wurde im Unterschied zu Vergleichsbeispiel 1 jedoch nicht mit Formiergas in der Gasphase, sondern in wäßriger Phase mit Hydrazin reduziert.

### Vergleichsbeispiel 3:

Es wurde ein Palladium-Gold-Kaliumacetat-Katalysator wie im Vergleichsbeispiel 1 beschrieben hergestellt. Als Katalysatorträger wurde der Alumosilikatträger KA 160 verwendet. Der Katalysator wurde im Unterschied zu Vergleichsbeispiel 1 jedoch nicht mit Formiergas, sondern mit Ethen in der Gasphase bei 150 °C reduziert.

### Beispiel 1: gemäß Erfindung

Es wurde ein Palladium-Gold-Kaliumacetat-Katalysator wie im Vergleichsbeispiel 1 hergestellt. Als Katalysatorträger wurde der Alumosilikatträger KA 160 verwendet. Der Katalysator wurde im Unterschied zu Vergleichsbeispiel 1 jedoch nicht bei 150 °C, sondern bei 450 °C in der Gasphase mit Formiergas reduziert.

### Anwendungsbeispiel 1

Aktivität und Selektivität der Katalysatoren aus den Vergleichsbeispielen 1, 2 und 3 und aus Beispiel 1 wurden während einer Prüfung über die Dauer von bis zu 24 Stunden gemessen.

Die Katalysatoren wurden in einem mit öl beheizten Strömungsrohrreaktor (Reaktorlänge 710 mm, Innendurchmesser 23,7 mm) bei Normaldruck und einer Raumgeschwindigkeit (GHSV) von 400 h⁻¹ mit der folgenden Gaszusammensetzung geprüft: 75 Vol.-% Ethen, 16,6 Vol.-% Essigsäure, 8,3 Vol.-% Sauerstoff. Die Katalysatoren wurden im Temperaturbereich von 120 bis 165 °C, gemessen im Katalysatorbett, untersucht.

Die Reaktionsprodukte wurden im Ausgang des Reaktors mittels on-line Gaschromatographie analysiert. Als Maß für die Katalysatoraktivität wurde die Raum-Zeit-Ausbeute des Katalysators in Gramm Vinyl'acetat-Monomer pro Stunde und Liter Katalysatorvolumen (g VAM/(h^{·}_{Kat.})) bzw. in Gramm Vinylacetat-Monomer pro Stunde und Kilogramm Katalysator (g VAM/(h^{·}kg_{Kat.})) bestimmt. Kohlendioxid, das insbesondere durch die Verbrennung von Ethen gebildet wird, wurde ebenfalls bestimmt und zur Beurteilung der Katalysatorselektivität herangezogen.

In Tabelle 2 sind die Untersuchungsergebnisse an den Katalysatoren aus den Vergleichsbeispielen 1, 2 und 3 sowie aus Beispiel 1 dargestellt.

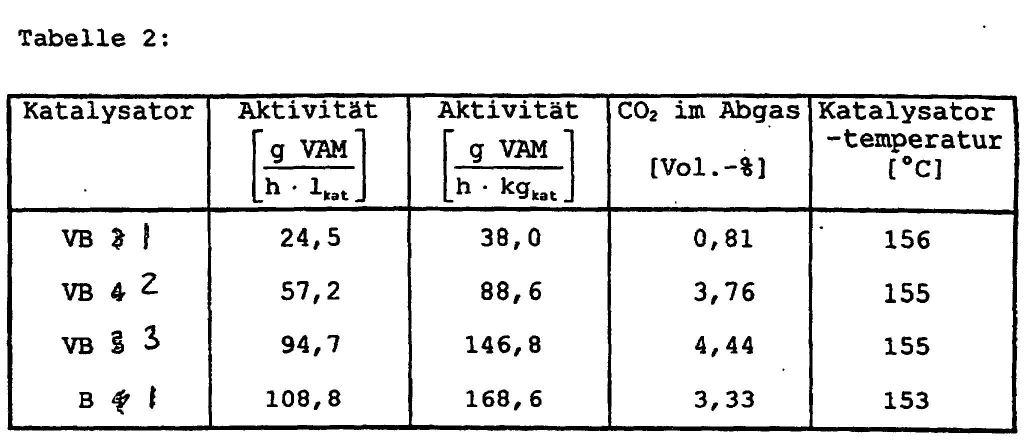

Das Beispiel 1 zeigt, daß der bei 450°C mit Formiergas reduzierte Katalysator eine deutlich gesteigerte Raum-Zeit-Ausbeute an Vinylacetatmonomer ermöglicht.

Wird der Katalysator wie im Vergleichsbeispiel 1 beschrieben bei 150 °C mit Formiergas in der Gasphase reduziert, so ergibt sich lediglich eine sehr geringe Raum-Zeit-Ausbeute an Vinylacetatmonomer. Die Reduktion in flüssiger Phase mit Hydrazin (Vergleichsbeispiel 2) bzw. mit Ethen in der Gasphase bei 150 °C (Vergleichsbeispiel 3) führt zu Katalysatoren mit deutlich verbesserter Raum-Zeit-Ausbeute an Vinylacetatmonomer. Wird der Katalysator nun wie im Beispiel 1 beschrieben bei 450 °C in der Gasphase mit Formiergas reduziert, so kann die Raum-Zeit-Ausbeute an Vinylacetatmonomer überraschenderweise nochmals deutlich gesteigert werden. Im Vergleich zu den weniger selektiven Katalysatoren aus den Vergleichsbeispielen 2 und 3 konnteauch die Selektivität deutlich gesteigert werden.

Je nach Aktivität und Selektivität der Katalysatoren wurde die Reaktortemperatur im Temperaturbereich von 130 - 145 °C so eingeregelt, daß die Temperatur in der Mitte des Katalysatorbettes zwischen 150 und 160 °C lag.

Die Reaktionsprodukte wurden im Ausgang des Reaktors kondensiert und mittels Gaschromatographie auf ihre Zusammensetzung untersucht. Als Maß für die Katalysatoraktivität wurde die Raum-Zeit-Ausbeute des Katalysators in Gramm Vinylacetatmonomer pro Stunde und Liter Katalysatorvolumen (g VAM/(h . l_{kat.})) beziehungsweise in Gramm Vinylacetatmonomer pro Stunde und Kilogramm Katalysator (g VAM/(h . kg_{kat.})) bestimmt. Kohlendioxid, welches insbesondere durch die Verbrennung von Ethen gebildet wird, wurde im Abgas des Reaktors gemessen und zur Beurteilung der Katalysatorselektivität herangezogen.

In Tabelle 1 sind die Testergebnisse der auf den vorbehandelten Katalysatorträgern hergestellten Katalysatoren B1, B2 und B3 im Vergleich zu den auf den nicht vorbehandelten Katalysatorträgern hergestellten Katalysatoren (Vergleichskatalysatoren) VB1 und VB2 dargestellt.

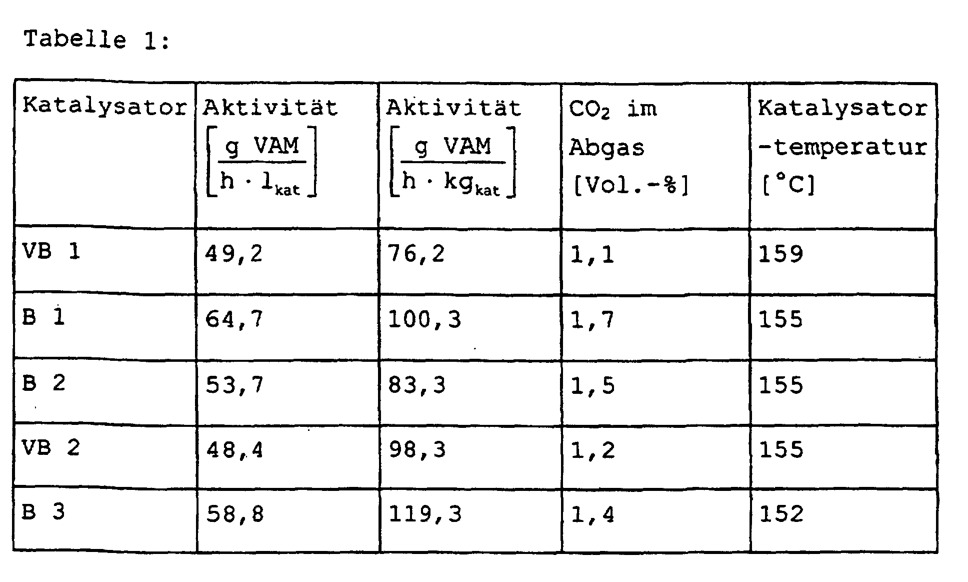

Die Beispiele zeigen, daß die erfindungsgemäßen Katalysatoren eine deutlich gesteigerte Raum-Zeit-Ausbeute an Vinylacetatmonomer bei vergleichbarer Bildung von CO₂ ermöglichen.

Der Katalysator auf dem Träger aus modifizierter pyrogener Kieselsäure (Beispiel 3) weist gewichtsbezogen eine deutlich höhere Raum-Zeit-Ausbeute auf als die Katalysatoren auf dem Träger aus modifiziertem Alumosilikat KA 160 (Beispiele 1 und 2). Der Katalysator nach Beispiel 3 ist darüberhinaus selektiver als der entsprechende Katalysator nach Beispiel 1.

### Vergleichsbeispiel 3:

Es wurde ein konventioneller Palladium-Gold-Kaliumacetat-Katalysator auf dem Alumosilikatträger KA 160 hergestellt. Die Konzentration der Imprägierlösungen wurde so gewählt, daß der fertige Katalysator 3,3 g Palladium, 1,5 g Gold und 30 g Kaliumacetat pro Liter Schüttvolumen des Katalysatorträgers enthielt. Dies entsprach einer Konzentration von 0,55 Gew.-% Palladium, 0,25 Gew.-% Gold und 5,0 Gew.-% Kaliumacetat bezogen auf das Gewicht des eingesetzten Trägers.

In einem ersten Schritt wurde der Träger zunächst mit einer basischen Lösung aus Natriumhydroxid in Wasser imprägniert. Das Volumen der wäßrigen NaOH-Lösung entsprach 50 Prozent der Wasseraufnahme des trockenen Trägers. Nach der Imprägnierung mit Natriumhydroxid wurde der Träger unmittelbar ohne Zwischentrocknung mit einer wäßrigen Edelmetallösung aus Natriumpalladiumchlorid und Tetrachlorogoldsäure imprägniert, deren Volumen ebenfalls 50 % der Wasseraufnahmekapazität des trockenen Trägermaterials entsprach.

Nach einer Wartezeit von 1,5 Stunden zwecks Hydrolyse der Edelmetallverbindungen wurden die Trägerteilchen chlorfrei gewaschen.

Der Katalysator wurde getrocknet und wie in der EP 0 634 209 beschrieben bei 150°C in der Gasphase mit Formiergas reduziert. Danach wurde der Katalysator mit einer wäßrigen Kaliumacetat-Lösung imprägniert und erneut getrocknet. Die Trocknung wurde in der Gasphase mit Stickstoff durchgeführt.

Die Konzentration der basischen Lösung an Natriumhydroxid war so bemessen, daß sich auf den Trägerteilchen eine edelmetallhaltige Schale von 0,3 mm Dicke ausbildete.

### Vergleichsbeispiel 4:

Es wurde ein Palladium-Gold-Kaliumacetat-Katalysator wie im Vergleichsbeispiel 3 beschrieben hergestellt. Als Katalysatorträger wurde der Alumosilikatträger KA 160 verwendet. Der Katalysator wurde im Unterschied zu Vergleichsbeispiel 2 jedoch nicht mit Formiergas in der Gasphase, sondern in wäßriger Phase mit Hydrazin reduziert.

### Vergleichsbeispiel 5:

Es wurde ein Palladium-Gold-Kaliumacetat-Katalysator wie im Vergleichsbeispiel 3 beschrieben hergestellt. Als Katalysatorträger wurde der Alumosilikatträger KA 160 verwendet. Der Katalysator wurde im Unterschied zu Vergleichsbeispiel 3 jedoch nicht mit Formiergas, sondern mit Ethen in der Gasphase bei 150 °C reduziert.

### Beispiel 4:

Es wurde ein Palladium-Gold-Kaliumacetat-Katalysator wie im Vergleichsbeispiel 3 hergestellt. Als Katalysatorträger wurde der Alumosilikatträger KA 160 verwendet. Der Katalysator wurde im Unterschied zu Vergleichsbeispiel 3 jedoch nicht bei 150 °C, sondern bei 450 °C in der Gasphase mit Formiergas reduziert.

### Anwendungsbeispiel 2

Aktivität und Selektivität der Katalysatoren aus den Vergleichsbeispielen 3, 4 und 5 und aus Beispiel 4 wurden während einer Prüfung über die Dauer von bis zu 24 Stunden gemessen.

Die Katalysatoren wurden in einem mit Öl beheizten Strömungsrohrreaktor (Reaktorlänge 710 mm, Innendurchmesser 23,7 mm) bei Normaldruck und einer Raumgeschwindigkeit (GHSV) von 400 h-1 mit der folgenden Gaszusammensetzung geprüft: 75 Vol.-% Ethen, 16,6 Vol.-% Essigsäure, 8,3 Vol.-% Sauerstoff. Die Katalysatoren wurden im Temperaturbereich von 120 bis 165 °C, gemessen im Katalysatorbett, untersucht.

Die Reaktionsprodukte wurden im Ausgang des Reaktors mittels on-line Gaschromatographie analysiert. Als Maß für die Katalysatoraktivität wurde die Raum-Zeit-Ausbeute des Katalysators in Gramm Vinylacetat-Monomer pro Stunde und Liter Katalysatorvolumen (g VAM/(h.l_{Kat}.)) beziehungsweise in Gramm Vinylacetat-Monomer pro Stunde und Kilogramm Katalysator (g VAM/(h.kg_{Kat}.)) bestimmt. Kohlendioxid, das insbesondere durch die Verbrennung von Ethen gebildet wird, wurde ebenfalls bestimmt und zur Beurteilung der Katalysatorselektivität herangezogen.

In Tabelle 2 sind die Untersuchungsergebnisse an den Katalysatoren aus den Vergleichsbeispielen 3, 4 und 5 sowie aus Beispiel 4 dargestellt.

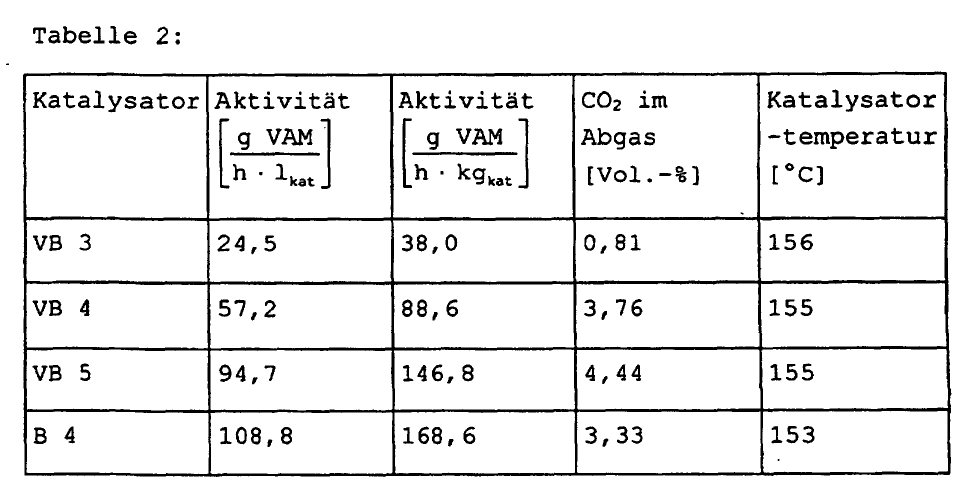

Das Beispiel 4 zeigt, daß der bei 450°C mit Formiergas reduzierte Katalysator eine deutlich gesteigerte Raum-Zeit-Ausbeute an Vinylacetatmonomer ermöglicht.

Wird der Katalysator wie im Vergleichsbeispiel 3 beschrieben bei 150 °C mit Formiergas in der Gasphase reduziert, so ergibt sich lediglich eine sehr geringe Raum-Zeit-Ausbeute an Vinylacetatmonomer. Die Reduktion in flüssiger Phase mit Hydrazin (Vergleichsbeispiel 4) beziehungsweise mit Ethen in der Gasphase bei 150 °C (Vergleichsbeispiel 5) führt zu Katalysatoren mit deutlich verbesserter Raum-Zeit-Ausbeute an Vinylacetatmonomer. Wird der Katalysator nun wie im Beispiel 4 beschrieben bei 450 °C in der Gasphase mit Formiergas reduziert, so kann die Raum-Zeit-Ausbeute an Vinylacetatmonomer überraschenderweise nochmals deutlich gesteigert werden. Im Vergleich zu den weniger selektiven Katalysatoren aus den Vergleichsbeispielen 4 und 5 konnte auch die Selektivität deutlich gesteigert werden.

### Vergleichsbeispiel 6:

Es wurde ein Palladium-Gold-Kaliumacetat-Katalysator auf dem Alumosilikatträger KA 160 wie im Beispiel 4 beschrieben hergestellt. Der Katalysatorträger wurde jedoch vorher mit Lanthannitrat imprägniert.

Hierzu wurde eine wäßrige Lösung aus Lanthannitrat angesetzt, deren Konzentration so bemessen war, daß 0,11 mol Lanthannitrat pro 200 g Trägermaterial vorlagen. Nach diesem Imprägnierschritt wurde der Katalysatorträger bei 150 - 180 °C für die Dauer von 2 Stunden getrocknet und calciniert.

### Vergleichsbeispiel 7:

Es wurde ein Palladium-Gold-Kaliumacetat-Katalysator auf dem Alumosilikatträger KA 160 wie im Vergleichsbeispiel 6 beschrieben hergestellt. Anstelle mit Lanthannitrat wurde der Katalysatorträger mit Wismutchlorid vorbehandelt.

### Beispiel 5:

Es wurde ein Palladium-Gold-Kaliumacetat-Katalysator auf dem Alumosilikatträger KA 160 wie im Vergleichsbeispiel 6 beschrieben hergestellt. Anstelle mit Lanthannitrat wurde der Katalysatorträger mit Titan(III)-chlorid vorbehandelt.

### Beispiel 6:

Es wurde ein Palladium-Gold-Kaliumacetat-Katalysator auf dem Alumosilikatträger KA 160 wie im Vergleichsbeispiel 6 beschrieben hergestellt. Anstelle mit Lanthannitrat wurde der Katalysatorträger mit Zirkoniumacetat vorbehandelt.

Die Katalysatoren der Beispiele B4 bis B6 und der Vergleichsbeispiele VB6 und VB7 wurden wie im Anwendungsbeispiel 2 beschrieben auf Aktivität und Selektivität geprüft. Die Untersuchungsergebnisse sind in Tabelle 3 zusammengestellt.

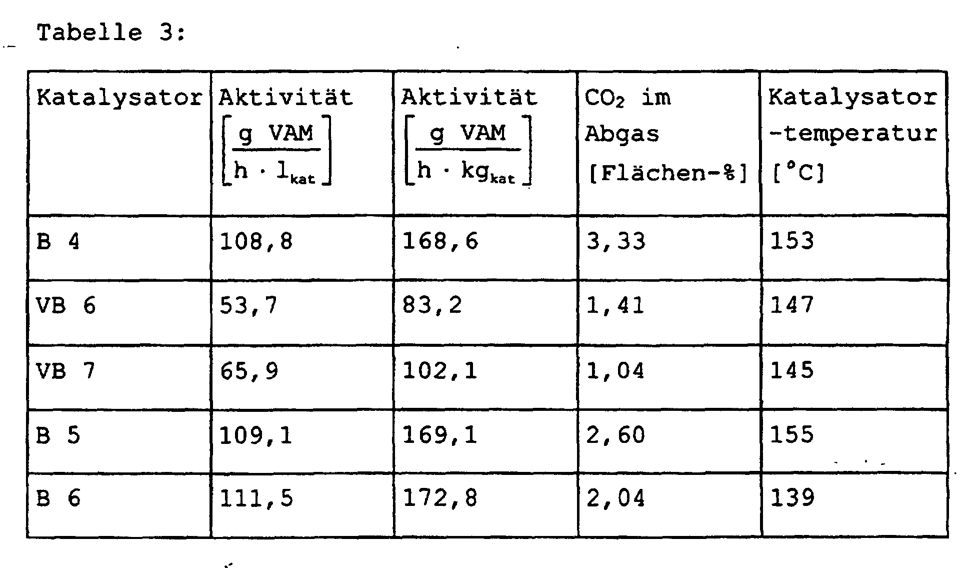

In Tabelle 3 sind die Katalysatortemperaturen angegeben, bei denen die Katalysatoren die jeweils höchste Raum-Zeit-Ausbeute ergaben.

Die Katalysatoren aus Vergleichsbeispiel 6 (Lanthan modifizierter Katalysatorträger) und aus Vergleichsbeispiel 7 (Wismut modifizierter Katalysatorträger) zeigen wesentlich schlechtere Leistungsdaten als die Katalysatoren der Beispiele B4 bis B6. Die Katalysatoren aus Beispiel 5 (Titan modifizierter Katalysatorträger) und aus Beispiel 6 (Zirkon modifizierter Katalysatorträger) sind noch etwas aktiver und selektiver als die Katalysatoren aus Beispiel 4 auf dem nicht modifizierten Katalysatorträger. Die Katalysatoren von Beispiel 6 (Zirkon modifizierter Katalysatorträger) ergeben die höchste Raum-Zeit-Ausbeute bei einer vergleichsweise sehr niedrigen Temperatur von 139 °C.

### Beispiel 7:

Es wurde ein Palladium-Gold-Kaliumacetat-Katalysator auf dem Alumosilikatträger KA 160 nach Beispiel 4 hergestellt.

Im Unterschied zum Katalysator nach Beispiel 4 wurde der Katalysator im vorliegenden Fall jedoch mit einem Edelmetallgehalt von 1,2 Gew.-% Palladium, 0,5 Gew.-% Gold und 5 Gew.-% Kaliumacetat hergestellt.

### Beispiel 8:

Es wurde ein Palladium-Gold-Kaliumacetat-Katalysator auf dem Alumosilikatträger KA 160 nach Beispiel 4 hergestellt.

Im Unterschied zum Katalysator nach Beispiel 4 wurde der Katalysator im vorliegenden Fall jedoch mit einem Edelmetallgehalt von 1,2 Gew.-% Palladium, 0,5 Gew.-% Gold und 5 Gew.-% Kaliumacetat hergestellt. Zusätzlich wurde der Katalysatorträger vor der Belegung mit Edelmetall mit Zirkoniumacetat wie in Beispiel 2 beschrieben imprägniert.

### Beispiel 9:

Es wurde ein Palladium-Gold-Kaliumacetat-Katalysator auf dem Alumosilikatträger KA 160 nach Beispiel 8 hergestellt.

Abweichend von Beispiel 8 wurde der Katalysatorträger mit Aluminiumchloridhydrat wie in Beispiel 1 beschrieben vorbehandelt.

In Tabelle 4 sind die Testergebnisse der auf den vorbehandelten beziehungsweise modifizierten Katalysatorträgern hergestellten Katalysatoren im Vergleich zu dem auf dem nicht vorbehandelten Katalysatorträger hergestellten Katalysator aus Beispiel 7 dargestellt. Die Katalysatoren wurden wie im Anwendungsbeispiel 2 beschrieben untersucht.

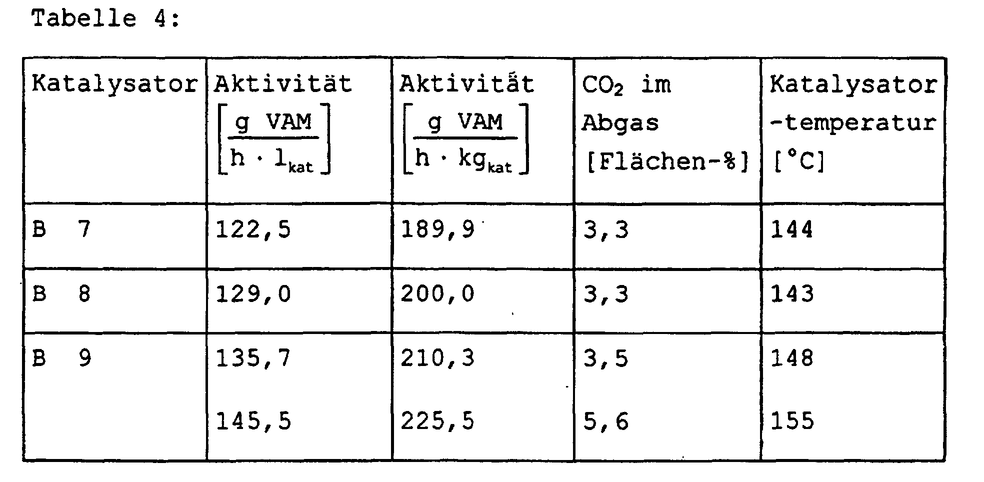

Auch die Beispiele 8 und 9 zeigen, daß Katalysatoren auf modifizierten Katalysatorträgern bei vergleichbarer Selektivität eine deutlich erhöhte Raum-Zeit-Ausbeute aufweisen.

### Beispiel 10:

Es wurde eine Palladium-Gold-Kaliumacetat Katalysator nach Beispiel 4 hergestellt. Als Katalysatorträger wurde ein Träger aus pyrogener Kieselsäure wie in Vergleichsbeispiel 2 eingesetzt. Im Unterschied zum Katalysator nach Beispiel 4 wurde der Katalysator in diesem Fall mit einem Edelmetallgehalt von 1,2 Gew.-% Palladium, 0,5 Gew.-% Gold und 5 Gew.-% Kaliumacetat hergestellt.

### Beispiel 11:

Es wurde ein Palladium-Gold-Kaliumacetat-Katalysator nach Beispiel 10 hergestellt.

Im Unterschied zum Beispiel 10 wurde der Katalysatorträger vor der Belegung mit Edelmetall mit Zirkoniumacetat wie in Beispiel 2 beschrieben imprägniert.

### Beispiel 12:

Es wurde ein Palladium-Gold-Kaliumacetat-Katalysator nach Beispiel 11 hergestellt.

Im Unterschied zum Katalysator nach Beispiel 11 wurde der Katalysatorträger nach der Vorbehandlung mit Zirkoniumacetat nicht bei 150 bis 180° C getrocknet und calciniert, sondern zunächst bei 120°C getrocknet und dann bei 400°C für die Dauer von 1 bis 2 Stunden calciniert.

In Tabelle 5 sind die Testergebnisse der auf den vorbehandelten Katalysatorträgern hergestellten Katalysatoren im Vergleich zu dem auf dem nicht vorbehandelten Katalysatorträger hergestellten Katalysator aus Beispiel 10 dargestellt. Die Katalysatoren wurden wie im Anwendungsbeispiel 2 beschrieben untersucht.

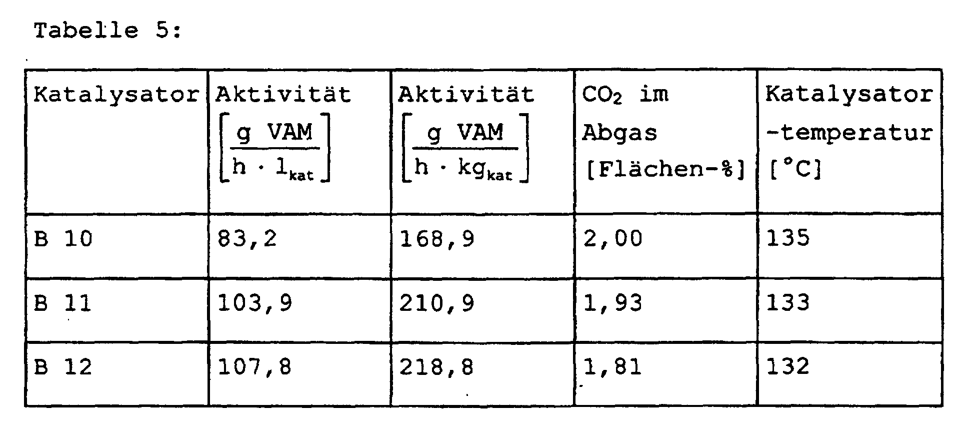

Die Katalysatortestergebnisse in Tabelle 5 zeigen, daß die Katalysatoren nach Beispiel 11 und 12 auf modifizierten Katalysatorträgern im Vergleich zu dem Katalysator aus Beispiel 10 auf einem nicht modifizierten Katalysatorträger sowohl eine deutlich höhere Aktivität als auch eine niedrigere CO₂-Bildung und damit eine höhere Selektivität aufweisen.

### Beispiel 13

In den folgenden Beispielen wurde der AEROSOL-Träger 350 von Degussa (siehe Vergleichsbeispiel 2) durch Imprägnieren mit verschiedenen Titan- und Zirkonverbindungen modifiziert.

Zur Modifizierung von 100 g Träger mit 1,5 Gew.-% Titan wurden 33 g einer 15 %igen Titanchlorid-Lösung (TiCl3) mit Wasser auf 80 ml entsprechend dem Porenvolumen des Trägermaterials verdünnt. Mit dieser Lösung wurde das Trägermaterial imprägniert.

Nach 30 Minuten Einwirkzeit wurde der Träger bei 100°C während 3 Stunden im Trockenschrank getrocknet und anschließend in einem Ofen bei 600°C für die Dauer von 4 Stunden calciniert.

### Beispiel 14

Zur Modifizierung von 100 g Träger mit 4 Gew.-% Titan wurden 85,93 g einer 15 %igen Titanchlorid-Lösung mit Wasser auf 80 ml verdünnt und zur Imprägnierung des Trägers über den Träger verteilt.

Nach 30 Minuten Einwirkzeit wurde der Träger bei 100°C während 3 Stunden im Trockenschrank getrocknet und anschließend in einem Ofen bei 600°C für die Dauer von 4 Stunden calciniert.

### Beispiel 15

Zur Modifizierung von 100 g Träger mit 5 Gew.-% Titan wurden 35,5 g Tetrabutoxytitan (Ti(C₄H₉O)₄) mit Butanol auf 80 ml verdünnt und über den Träger verteilt.

Nach 30 Minuten Einwirkzeit wurde der Träger bei 100°C während 3 Stunden im Trockenschrank getrocknet und anschließend in einem Ofen bei 600°C für die Dauer von 4 Stunden calciniert.

### Beispiel 16

Zur Modifizierung von 100 g Träger mit 5 Gew.-% Zirkon wurden 21,03 g Tetrabutoxyzirkon (IV) mit Butanol auf 80 ml verdünnt und über den Träger verteilt.

Nach 30 Minuten Einwirkzeit wurde der Träger bei 100°C während 3 Stunden im Trockenschrank getrocknet und anschließend in einem Ofen bei 600°C für die Dauer von 4 Stunden calciniert.

### Beispiel 17

Zur Modifizierung von 100 g Träger mit 8 Gew.-% Zirkon wurden 28,23 g Zirkonylchlorid (ZrOCl₂ · 8H₂O) in 62,37 g Wasser gelöst und über den Träger verteilt.

Nach 30 Minuten Einwirkzeit wurde der Träger über Nacht im Trockenschrank bei 120°C getrocknet und anschließend in einem Ofen bei 500°C für die Dauer von 3 Stunden calciniert.

Die Druckfestigkeit der Trägerteilchen betrug vor der Modifizierung 101 N (radiale Messung). Nach der Modifizierung mit 8 Gew.-% Zirkon betrug die Druckfestigkeit 140 N.

### Beispiel 18

Es wurden 100 g Träger wie in Beispiel 17 mit 8 Gew.-% Zirkon modifiziert. Zur Untersuchung des Einflusses der Calciniertemperatur auf die Erhöhung der Druckfestigkeit der Trägerteilchen wurde ein Drittel der Trägerteilchen 3 Stunden bei 200, ein weiteres Drittel 3 Stunden bei 400 und das letzte Drittel 3 Stunden bei 600°C calciniert.

Die Druckfestigkeit dieser Trägerteilchen betrugen 123 N (Calcinierung bei 200°C), 153 N (Calcinierung bei 400°C) und 148 N (Calcinierung bei 600°C).

### Beispiel 19

Der Zirkongehalt des Trägers von Beispiel 17 sollte durch eine erneute Imprägnierung weiter erhöht werden. Hierzu wurden 8,9 g Zirkonylchlorid (ZrOCl₂ · 8H₂O) in 7,23 g Wasser gelöst und damit 14,95 g des Trägers imprägniert. Dadurch erhöhte sich der Zirkongehalt des Trägers auf 21,1 Gew.-%.

Nach 30 Minuten Einwirkzeit wurde der Träger über Nacht im Trockenschrank bei 120°C getrocknet und anschließend im Ofen bei 500°C für die Dauer von 3 Stunden calciniert.

Die Druckfestigkeit der mit 21,1 Gew.-% modifizierten Trägerteilchen betrug 163 N.

## Patentansprüche

1. Trägerkatalysator für die Produktion von Vinylacetatmonomer enthaltend auf einem Träger aus Siliziumdioxid, Alumosilikat oder Aluminiumoxid als katalytisch aktive Komponenten Palladium, Gold und Alkaliacetat, erhältlich durch Imprägnieren des Trägers mit einer basischen Lösung, einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile, und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln,
**dadurch gekennzeichnet,**
**daß** die Reduktion im Formiergas (95 Vol. % N₂ + 5 Vol. % H₂) bei Temperaturen zwischen 300 und 550, bevorzugt zwischen 350 und 500°C durchgeführt wird.

2. Verfahren zur Herstellung eines Trägerkatalysators für die Produktion von Vinylacetatmonomer enthaltend auf einem Träger aus Siliziumdioxid, Alumosilikat oder Aluminiumoxid als katalytisch aktive Komponenten Palladium, Gold und Alkaliacetat durch Imprägnieren des Trägers mit einer basischen Lösung, einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile, und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln,
**dadurch gekennzeichnet,**
**daß** die Reduktion im Formiergas (95 Vol. % N₂ + 5 Vol. % H₂) bei Temperaturen zwischen 300 und 550, bevorzugt zwischen 350 und 500°C durchgeführt wird.

3. Verwendung der Katalysatoren nach einem der vorstehenden Ansprüche für die Produktion von Vinylacetatmonomer durch Umsetzung von Ethylen, Essigsäure und Sauerstoff an dem Katalysator in der Gasphase bei erhöhtem Druck.

## Claims

1. Support catalyst for the production of vinyl acetate monomer, containing, on a support of silicon dioxide, of alumosilicate or of aluminium oxide, palladium, gold and alkali acetate as the catalytically active components, obtainable by impregnating the support with a basic solution and with a solution containing gold salts and palladium salts, the impregnations being carried out simultaneously or in succession, with or without intermediate drying, washing the support in order to remove any chloride components that may be present and, before or after the washing step, reducing the insoluble compounds that have precipitated on the support, drying the catalyst precursor so obtained, and impregnating it with alkali acetates or with alkali compounds that are converted wholly or partially into alkali acetates under the reaction conditions in the production of vinyl acetate monomer,
**characterised in that**
the reduction is carried out in forming gas (95 vol.% N₂ + 5 vol.% H₂) at temperatures from 300 to 550°C, preferably from 350 to 500°C.

2. Process for the preparation of a support catalyst for the production of vinyl acetate monomer, containing, on a support of silicon dioxide, of alumosilicate or of aluminium oxide, palladium, gold and alkali acetate as the catalytically active components, by impregnating the support with a basic solution and with a solution containing gold salts and palladium salts, the impregnations being carried out simultaneously or in succession, with or without intermediate drying, washing the support in order to remove any chloride components that may be present and, before or after the washing step, reducing the insoluble compounds that have precipitated on the support, drying the catalyst precursor so obtained,. and impregnating it with alkali acetates or with alkali compounds that are converted wholly or partially into alkali acetates under the reaction conditions in the production of vinyl acetate monomer,
**characterised in that**
the reduction is carried out in forming gas (95 vol.% N₂ + 5 vol.% H₂) at temperatures from 300 to 550°C, preferably from 350 to 500°C.

3. Use of catalysts according to any one of the preceding claims in the production of vinyl acetate monomer by reaction of ethylene, acetic acid and oxygen on the catalyst in the gas phase at elevated pressure.

## Revendications

1. Catalyseur sur support pour la production de monomère d'acétate de vinyle, contenant sur un support en dioxyde de silicium, d'aluminosilicate ou en oxyde d'aluminium, en tant que composants actifs catalytiquement du palladium, de l'or et un acétate de métal alcalin, accessible par imprégnation du support avec une solution alcaline, une solution contenant des sels d'or et des sels de palladium, pour lequel l'imprégnation s'effectue simultanément ou successivement, avec ou sans séchage intermédiaire, lavage du support pour éliminer les fractions de chlorure éventuellement présentes, et réduction des composés insolubles précipités sur le support avant ou après le lavage, séchage du stade précurseur de catalyseur ainsi obtenu, et imprégnation avec un acétate de métal alcalin ou avec des composés de métal alcalin, qui se transforment dans les conditions réactionnelles, au cours de la production de monomère d'acétate de vinyle, partiellement ou totalement en acétate de métal alcalin,
**caractérisé en ce que**
la réduction dans le gaz de formage (95 % en volume de N₂ et 5 % en volume de H₂) s'effectue à des températures comprises entre 300 et 550, de préférence entre 350 et 500°C.

2. Procédé de production d'un catalyseur sur support pour la production de monomère d'acétate de vinyle contenant sur un support en dioxyde de silicium, d'aluminosilicate ou en oxyde d'aluminium, comme composants actifs catalytiquement du palladium, de l'or et un acétate de métal alcalin, par imprégnation du support avec une solution basique, avec une solution contenant un sel de palladium et un sel d'or pour laquelle l'imprégnation s'effectue simultanément ou successivement, avec ou sans séchage intermédiaire, lavage du support en vue d'éliminer les fractions de chlorure éventuellement présentes, et réduction des composés insolubles précipités sur le support avant ou après le lavage, séchage du stade préliminaire de catalyseur ainsi obtenu, et imprégnation avec des acétates de métal alcalin ou des composés de métal alcalin qui se transforment dans les conditions de réaction au cours de la production de monomère d'acétate de vinyle, partiellement ou totalement en acétate de métal alcalin,
**caractérisé en ce que**
la réduction dans le gaz de formage (95 % en volume de N₂ et 5 % en volume de H₂) s'effectue à des températures comprises entre 300 et 550°C, de préférence entre 350 et 500°C.

3. Utilisation des catalyseurs selon l'une quelconque des revendications précédentes, pour la production de monomère d'acétate de vinyle, par réaction de l'éthylène, de l'acide acétique et de l'oxygène sur le catalyseur en phase gazeuse à pression accrue.
